(19) Europäisches Patentamt
European Patent Office
Office européen des brevets

(11) Numéro de publication: **0 262 000 B1**

(12) # FASCICULE DE BREVET EUROPEEN

(45) Date de publication de fascicule du brevet: **23.09.92** (51) Int. Cl.⁵: **A23L 3/34**

(21) Numéro de dépôt: **87401513.4**

(22) Date de dépôt: **30.06.87**

(54) **Procédé de régulation et contrôle de fermentations mixtes, aérobies et/ou anaérobies; son utilisation dans la fabrication ou la conservation de produits alimentaires.**

(30) Priorité: **30.06.86 FR 8609508**

(43) Date de publication de la demande:
**30.03.88 Bulletin 88/13**

(45) Mention de la délivrance du brevet:
**23.09.92 Bulletin 92/39**

(84) Etats contractants désignés:
**AT BE CH DE ES FR GB GR IT LI LU NL SE**

(56) Documents cités:

**DERWENT JAPANESE PATENT GAZETTE, vol. 9, no. 160, section C no. 289, juillet 1985, page 52**

**F.S.T.A. JOURNAL, réf. 74028920; P. STATICESCU et al.: "Effect of physiologically active substances in reducing sugar losses during sugar beet storage", & INST. DE CERCETARI SI PROIECTARE ALIMENTARE, BUCHAREST, RUMANIA, INDUSTRIA ALIMENTARA, vol. 23, no. 10, pages 540-544,552, 26 réf. 1972**

(73) Titulaire: **NUTRIGEN INTERNATIONAL S.A.**
**Rue Notre Dame 37**
**Luxembourg(LU)**

(72) Inventeur: **Bruyneel, Bart**
**Kerkstraat 9**
**B-9220 Merelbeke(BE)**
Inventeur: **Vande Woestijne, Marleen**
**Doornkapellestraat 11**
**B-8670 Wervik(BE)**
Inventeur: **Verstraete, Willy**
**Dasstraat 4**
**B-9030 Gent(BE)**

(74) Mandataire: **Peaucelle, Chantal et al**
**S.C. Ernest Gutmann - Yves Plasseraud 67, boulevard Haussmann**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 262 000 B1

F.S.T.A. JOURNAL, réf. 69041352; O. COLAGRANDE et al.: "Determination of ferrous and total iron in wine with 4,7-diphenyl-1,10-phenanthroline", & INSTITUTO DI INDUSTRIE AGRARIE, UNIV. CATTOLICA DEL S. CUORE, PIACENZA, ITALY, vol. 7, no. 5, pages 206-09, 14 réf., 1969

DERWENT JAPANESE PATENT GAZETTE, vol. 10, no. 219, section C no. 363, 31 juillet 1986, page 107

## Description

La présente invention a pour objet un nouveau procédé pour la régulation et le contrôle de fermentations mixtes aérobies et/ou anaérobies dans des produits industriels mettant en jeu des produits organiques, dans lesquels des fermentations, soit se produisent spontanément et qu'il importe de contrôler, soit sont provoquées par l'intermédiaire des ferments lactiques.

Elle concerne plus particulièrement l'utilisation de ce procédé, notamment dans les industries de la fabrication et/ou de la conservation de produits alimentaires.

Par composition ou produit alimentaire, on entend dans ce qui suit tout produit naturel ou ayant fait l'objet de transformations, notamment en vue d'accroître ses propriétés nutritives, et destiné à l'alimentation de l'homme ou de l'animal.

Entrent dans cette catégorie, à titre d'exemples, les produits laitiers (beurre, yaourts, crême), les produits fromagers, les produits d'alimentation du bétail (tel que fourrages ensilés). Entrent également dans le cadre de la définition ci-dessus, les boissons fermentées telles que vins, bières, cidre, ainsi que les produits alimentaires à base de légumes fermentés.

Beaucoup de fabrications d'aliments font intervenir des fermentations lactiques. Ces fermentations lactiques jouent par exemple un rôle important dans la fabrication des produits laitiers et fromagers. Elles jouent également un rôle dans la saveur et l'arôme des produits alimentaires.

Les industries alimentaires sont aussi confrontées à un problème majeur : assurer aux produits qu'elles fabriquent ou commercialisent un état de conservation tel qu'il autorise leur consommation sans aucun danger pour l'homme ou l'animal.

Les fermentations lactiques jouent également un rôle important à ce niveau. Elles interviennent par exemple au niveau de la conservation de nombreuses denrées alimentaires, notamment végetales : olives, cornichons.

Les fermentations lactiques interviennent également dans la conservation des fourrages ensilés qui nécessite d'être réalisée dans un milieu acide. La production d'acide lactique résultant de la fermentation lactique permet, dans ce cas, d'abaisser le pH à un niveau suffisamment bas pour assurer une bonne conservation des fourrages ensilés.

Mais il est bien connu que la fermentation lactique se développe souvent conjointement avec d'autres fermentations, par exemple les fermentations acétiques, butyriques, alcooliques, en milieu anaérobie et/ou micro-aérophile.

De tels systèmes de fermentations, appelés fermentations mixtes, sont responsables de nombreux accidents dans la fabrication ou la conservation de compositions ou produits alimentaires.

Ainsi, dans les industries laitières ou fromagères, la fermentation butyrique et la présence de Clostridia présentent un caractère nocif qu'il faut éliminer.

De même, la fermentation acétique dans les boissons fermentées entraîne des accidents dans la consommation de ces produits.

Ces fermentations parasites ont, en outre, l'effet très nuisible que représente leur substitution aux fermentations lactiques qui, elles, sont recherchées. Il suffit, en effet, que les bactéries lactiques se trouvent en présence de bactéries appartenant à des espèces différentes qui aient des capacités de résistance plus grande et de développement plus rapide pour que les fermentations lactiques recherchées ne puissent même plus s'effectuer.

Ces divers inconvénients entraînent de ce fait des pertes non négligeables dans les unités de production.

L'invention a pour but de remédier en grande partie à ces divers inconvénients, plus particulièrement de régler et contrôler à volonté les fermentations mixtes aérobies et/ou anaérobies dans des produits ou compositions, notamment alimentaires, ce réglage et ce contrôle visant à favoriser, ainsi qu'à réguler, les fermentations lactiques au détriment des fermentations non lactiques, et a, plus particulièrement, pour objet même d'inhiber à volonté ces fermentations non lactiques.

Le procédé de l'invention repose sur l'idée que les Inventeurs ont eu de rendre le fer, présent dans les compositions ou produits, notamment alimentaires, inaccessible aux micro-organismes également présents dans ces compositions ou produits et, partant, de neutraliser le développement de ceux de ces micro-organismes dont le développement est directement tributaire de la présence de fer dans le milieu environnant.

L'invention tire plus particulièrement profit de la qualité que possède le fer sous forme ionique de jouer un rôle essentiel dans le métabolisme des organismes vivants.

Las plupart des organismes vivants ont besoin de fer pour leur développment (Réf. 2). Cette nécessité de la présence de fer, sous forme d'ions ferreux $Fe^{++}$ ou d'ions ferriques $Fe^{+++}$, a notamment été montrée

chez Clostridia (réf. 3), Pseudomonas aeruginosa, Corynebac terium diphtheriae, Escherichia Coli, Ferrobacillus ferroxidans (Réf. 4).

Le document JP-A-60 34 158 décrit un procédé mettant en oeuvre une incorporation d'EDTA disodique et/ou calcique pour complexer au sein d'une composition alimentaire l'ensemble des composants métalliques formant des réserves nutritives pour des micro-organismes.

L'invention met à profit l'exception que représente la propriété qu'ont les bactéries lactiques, que l'on avait certes déjà mentionnée à titre de curiosité de laboratoire (Réf. 1), de se développer et de synthétiser de l'acide lactique dans un milieu dépourvu de fer.

Il était connu, d'autre part, que certains produits chimiques, ci-après désignés, par l'expression "chélateurs de fer", sont capables de former sélectivement avec les ions ferreux $Fe^{++}$ ou ferriques $Fe^{+++}$ des complexes stables et solubles.

Certains produits chimiques synthétiques possédant la propriété de chelater les ions ferreux ou ferriques étaient également connus.

Le procédé de régulation et/ou de contrôle de fermentations mixtes anaérobies et/ou aérobies, au sein d'une composition contenant des bactéries lactiques, caractérisé par l'incorporation et le maintien au sein de la composition d'un produit choisi pour sa capacité à complexer spécifiquement le fer, notamment à l'état d'ions ferreux $Fe^{++}$ ou ferrique $Fe^{+++}$, et ce pour favoriser la fermentation lactique et, simultanément, éviter le développement au sein de ladite composition d'autres organismes pour lesquels le fer constitue un élément vital, le produit complexant du fer étant présent en une dose fonction du degré voulu de chélation du fer présent.

La présence du fer permet donc le contrôle des fermentations non lactiques dès lors qu'une telle fermentation se produirait au sein de la composition, notamment alimentaire. Il va sans dire que cet effet sera également obtenu dans le cas où la fermentation sera provoquée, que ce soit aux fins d'une conservation ou d'une transformation de compositions initiales en vue de les transformer en des produits finaux désirés, par exemple dans le cas des produits laitiers ou fromagers.

Or il était connu que certains produits chimiques synthétiques, voire même certains produits secrétés par des micro-organismes déterminés possédaient la propriété de complexer ou de chélater des ions ferreux pour former des complexes stables et solubles de ces ions ferreux. Naturellement pour l'utilisation dans des compositions alimentaires, il faudra avoir recours à des produits dépourvus de toute toxicité, en tous les cas aux doses auxquelles ils sont susceptibles d'être utilisés.

Parmi les composés connus pour chélater le fer à l'état d'ions ferreux on mentionnera tout particulièrement le 2-2'-dipyridyl dont la capacité de complexer spécifiquement le fer a été utilisée en chimie analytique comme indicateur de la présence du fer dans une solution. D'autres produits présentent également des propriétes semblables. On mentionnera, à titre d'exemple, la bathophénantroline et la 1, 10 -phénantroline, ainsi que des produits naturels secrétés par des microorganismes tel que la sidérocholine A ou B (secrétée par Nocardia sp.) et la ferrorosamine (secrétée par Pseudomonas sp.). Ceci étant, et dans l'état actuel des recherches des Inventeurs, le 2-2'-dipyridyl est le composé préféré qui peut être mis en oeuvre dans le procédé de l'invention.

L'invention permet une meilleure qualité de fabrication ou de conservation de produits mettant en jeu des produits organiques dans lesquels se produisent des fermentations, notamment de produits alimentaires, en intervenant sur le paramètre que représente la présence de fer dans le milieu de fermentation du produit.

Les produits complexant ou chélatant le fer se sont en effet révélés rendre le fer indisponible dans les milieux particulièrement complexes que constituent les compositions alimentaires. Il en résulte que la croissance des micro-organismes dont le métabolisme requiert la présence de fer est alors inhibée dans lesdites compositions.

Bien que le 2-2'-dipyridyl représente l'agent préféré pour réaliser la complexation du fer, il va de soi que l'invention ne se limite pas à l'emploi de ce seul produit.

A titre d'exemples non limitatifs, on citera comme autres produits pouvant être utilisés la ferrorosamine, la bathophénantroline, la 1,10-phénantroline, la sidérocholine A ou B.

Tous les produits énumérés ci-dessus présentent un certain caractère de toxicité. Pour le cas où ils sont utilisés, selon le procédé de l'invention, dans les industries de fabrication et/ou de conservation de produits alimentaires, il convient d'abaisser la toxicité à un niveau tel qu'elle ne présente plus aucun danger pour l'homme ou l'animal.

On y parvient, par exemple, en ajoutant du fer dans les produits préparés, après que les processus de fermentation soient terminés et que l'on ait obtenu le produit final désiré et stable.

Plus généralement, toute substance capable de former avec un haut degré de spécificité des complexes stables avec les ions ferreux ou ferriques, peut être utilisée avec avantage dans le procédé de

l'invention, à condition toutefois qu'elle ne présente pas d'effets toxiques ou néfastes pour les produits alimentaires eux-mêmes ou pour le consommateur de ces produits.

Si les produits énumérés ci-dessus complexent essentiellement le fer, il n'en reste pas moins vrai que d'autres ions métalliques sont chélatés en même temps que le fer.

Par exemple, le 2-2'-dipyridyl complexe, en plus des ions ferreux $Fe^{++}$, les ions $Cu^{++}$, $Zn^{++}$ et $Mn^{++}$.

Il convient donc de choisir un produit dont la faculté de chélation des ions s'applique essentiellement sur le fer.

Le procédé de l'invention est particulièrement avantageux dans le cas des fermentations mixtes dont il a été question plus haut.

Dans de telles conditions anaérobies et/ou micro-aérophiles, le fer est présent principalement à l'état d'ions ferreux $Fe^{++}$.

Le procédé de l'invention permet donc dans les compositions, notamment alimentaires, d'inhiber le développement de tous les micro-organismes anaérobies et/ou micro-aérophiles, notamment du type Clostridia, à l'exception des bactéries lactiques ou lactobactéries dans le métabolisme desquelles le fer ne constitue pas un élément vital.

Le procédé de l'invention permet donc d'orienter et de réguler les processus de fermentations mixtes vers la seule fermentation lactique et d'éviter, de ce fait, le développement de micro-organismes, notamment de Clostridia et d'acétobactéries, responsables d'accidents dans la fabrication et la conservation de compositions ou produits alimentaires.

En outre la fermentation lactique contrôlée et régulée par le procédé de l'invention présente l'important avantage d'augmenter la quantité d'acide lacti que produit, et par voie de conséquence d'abaisser le pH à la valeur voulue et sous l'effet essentiel de l'acide lactique.

Cet aspect est particulièrement avantageux pour la conservation des aliments pour bétail, notamment les fourrages ensilés, qui doivent être conservés à un pH acide, d'environ 4.

Si l'utilisation du procéde de l'invention revêt un avantage particulier pour les fermentations anaérobies, il n'en reste pas moins vrai que l'invention s'applique également dans des conditions aérobies.

Dans un tel milieu aérobie, le fer est présent sous la forme d'ions ferreux $Fe^{++}$ et d'ions ferriques $Fe^{+++}$, qui sont en équilibre. La présence, dans ce milieu, d'un produit complexant les ions $Fe^{++}$ entraîne un déplacement de l'équilibre entraînant une réduction des ions ferriques $fe^{+++}$ en ions ferreux $Fe^{++}$. Il en résulte qu'à mesure que les ions $Fe^{++}$ sont complexés, la concentration du milieu en ions ferriques diminue. En ajoutant au milieu une concentration suffisante en produit complexant, on peut aboutir à la chélation de la quasi-totalité des ions $Fe^{++}$ et $Fe^{+++}$, après transformation de ces derniers en ions $Fe^{++}$.

Le procédé de l'invention, utilisant un produit chélatant les ions ferreux $Fe^{++}$, est donc également fonctionnel en milieu aérobie, ce qui est effectivement le plus souvent le cas.

Dans cette variante de mise en oeuvre de l'invention, l'addition d'une substance complexant le fer entraîne l'inhibition de la croissance de micro-organismes tels que bactéries, levures et champignons.

On dipose ainsi d'un moyen simple et efficace pour stabiliser et conserver les compositions ou produits alimentaires à l'abri des dégradations induites par ces organismes.

On peut également complexer directement le fer sous forme d'ions ferriques $Fe^{+++}$ sans passer par la réduction des ions $Fe^{+++}$ en $Fe^{++}$.

On y parvient en utilisant un produit complexant spécifiquement le fer sous forme d'ions $Fe^{+++}$.

A titre d'exemples non limitatifs, on citera comme produits utilisables, des produits naturels tels que la ferrioxamine B, le ferrichrome (réf. 5), l'entérobactine, la pseudobactine, de formule (I) ci-après (réf. 6)

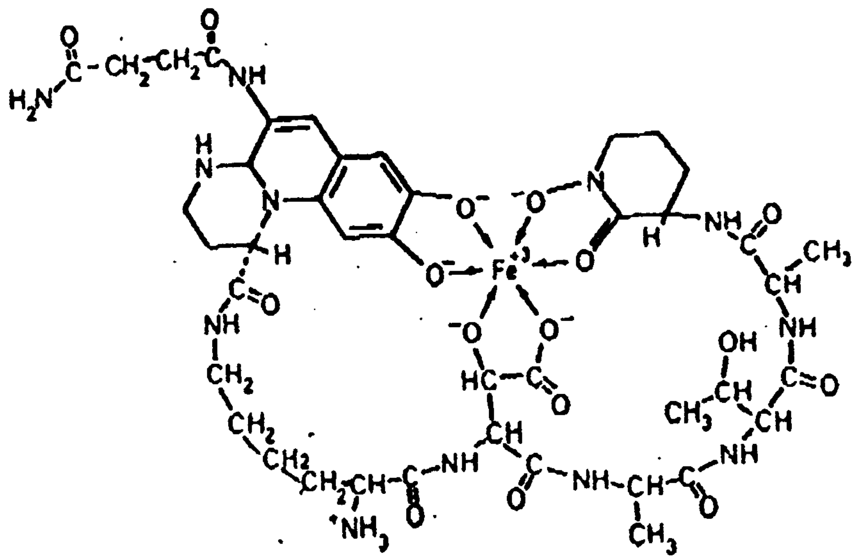

(I)

ou des produits synthétiques tels que l'EDDA (de l'anglais : "Ethylen-Diamin-Di-Acetic acid), la 8-hydroxyquinoline.

D'autres produits naturels utilisables en tant qu'agents chélateurs du fer ferrique $Fe^{+++}$ comprennent notamment ceux appartenant à la famille des phénolates-catécholates, tels que l'entérochéline de formule (II) ci-après (réf. 7) :

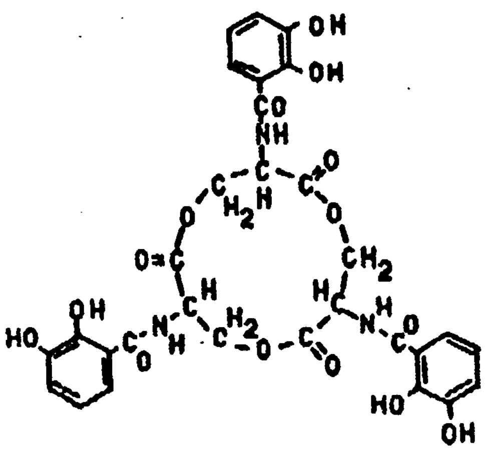

(II)

D'autres agents naturels chélateurs du fer ferrique $Fe^{+++}$ sont constitués par ceux appartenant à la famille des dihydroxamates de formule générale (III) ci-après :

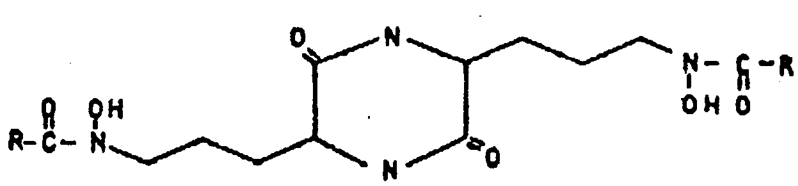

(III)

tels que notamment l'acide rhodoturulique (où R est un groupe méthyle) et l'acide démérumique (où R est

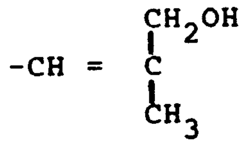

(réf. 8) ;

Une autre famille de produits naturels utilisables et celle des trihydroxamates, tels que notamment les ferrichromes de formule générale (IV) ci-après (réf. 5) :

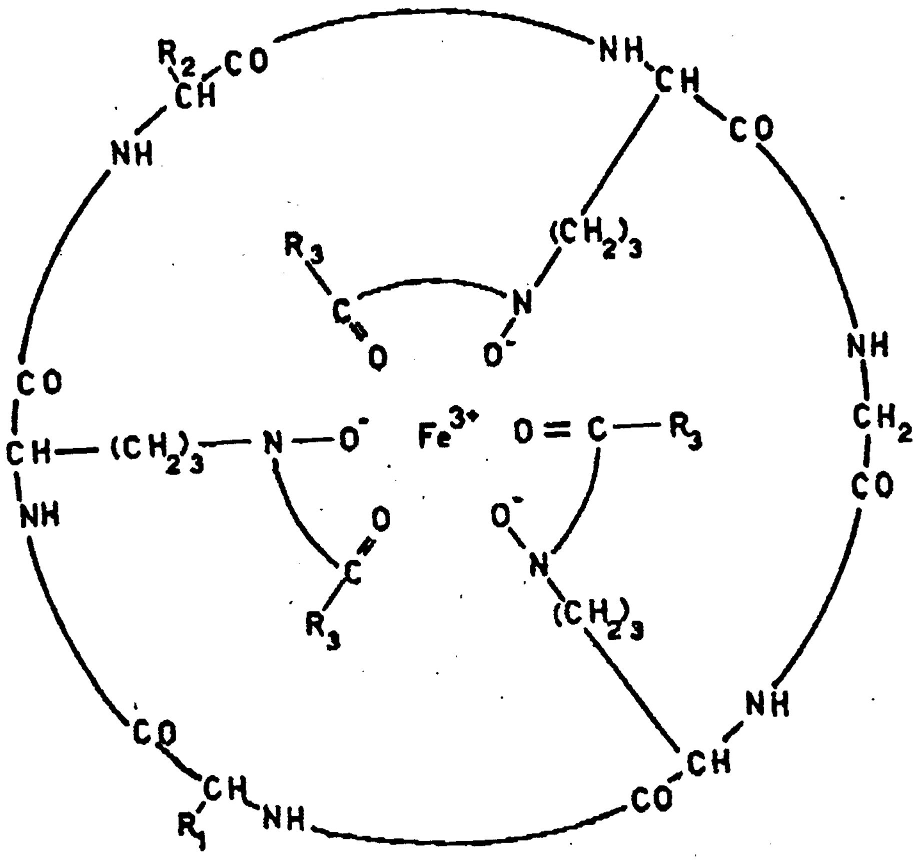

(IV)

Des ferrichromes préférés sont :
le ferrichrome où $R_1$ = -H, $R_2$ = -H, $R_3$ = $-CH_3$
la ferricrocine où $R_1$ = -H. $R_2$ = $-CH_2OH$, $R_3$ = $-CH_3$
la ferrichrysine où $R_1$ = $-CH_2OH$, $R_2$ = $-CH_2OH$, $R_3$ : $-CH_3$
la ferrirhodine où $R_1$ = $-CH_2OH$, $R_2$ = $-CH_2OH$,

$R_3$ =

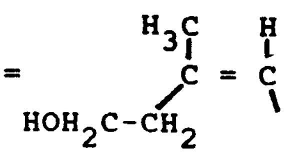

la ferrirubine où $R_1$ = $-CH_2OH$, $R_2$ = $-CH_2OH$,

$R_3$ = 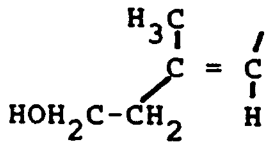

le ferrichrome A où $R_1$ = $-CH_2OH$, $R_2$ = $-CH_2OH$,

$R_3$ = 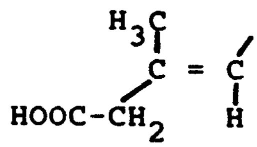

D'autres trihydroxamates utilisables sont constitués par ceux possédant un motif acide rhodoturilique et répondant à la formule générale (V) ci-après :

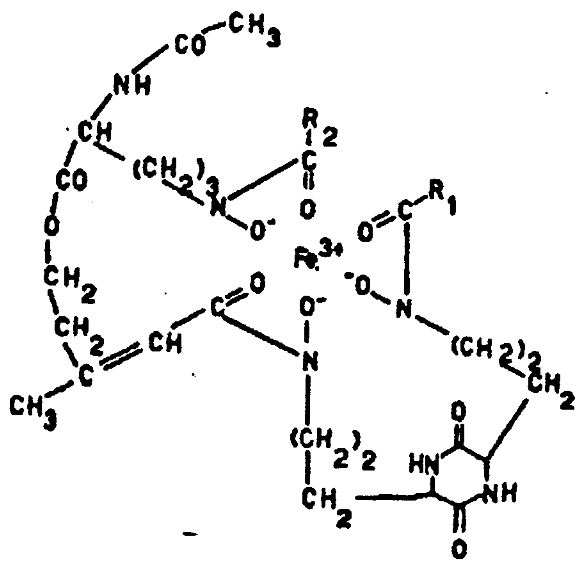

(V)

Appartiennent notamment à ce groupe :
le coprogène où

$$R_1 = -CH = \underset{CH_3}{\underset{|}{C}}-CH_2-CH_2-OH,\ R_2 = -HC = \underset{CH_3}{\underset{|}{C}}-CH_2-CH_2-OH \text{ (réf.9)}$$

la triornicine où

$$R_1 = -CH = C-\underset{CH_3}{\underset{|}{CH_2}}-CH_2-OH,\ R_2 = -CH_3, \qquad \text{(réf. 10)}$$

la isotriornicine où

$R_1 = -CH_3,\ R_2 = -CH = \underset{CH_3}{\underset{|}{C}}-CH_2-CH_2-OH$ (réf. 11)

Des produits naturels utilisables appartenant à la famille des trihydroxamates sont encore constitués par les fusarinines de formule générale (VI) ci-après (réf. 9) :

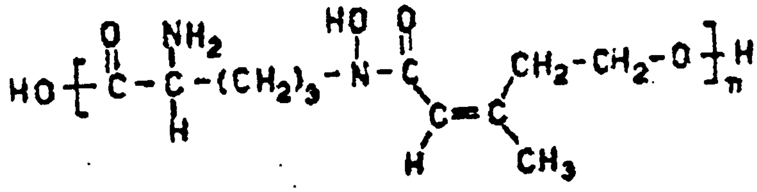

(VI)

et les ferrioxamines, de formule générale (VII) ci-après (réf. 9) :

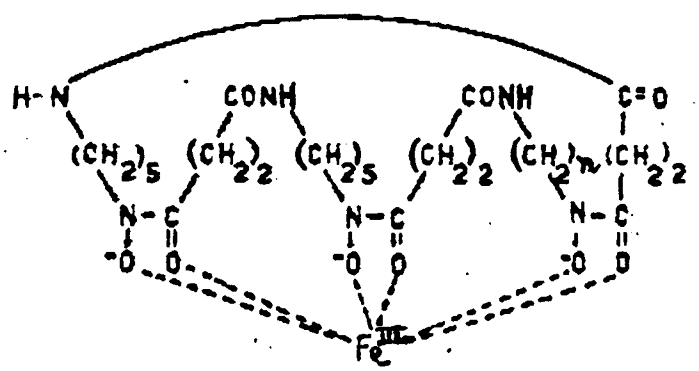

(VII)

D'autres produits naturels utilisables sont ceux répondant à la formule générale (VIII) ci-après :

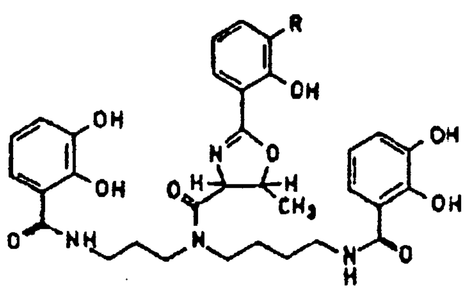

(VIII)

parmi lesquels figure la parabactine (où R = -H) et l'agrobactine (où R = -OH) (réf. 9).

La mycobactine (réf. 12) de formule (IX) ci-après est également utilisable comme agent chélateur naturel du fer ferrique $Fe^{+++}$.

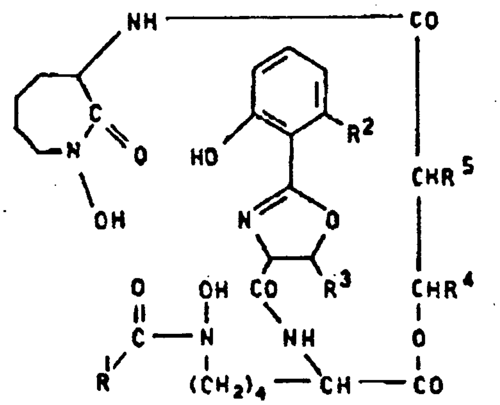

(IX)

D'autres produits naturels utilisables sont encore ceux répondant à la formule générale (X) ci-après :

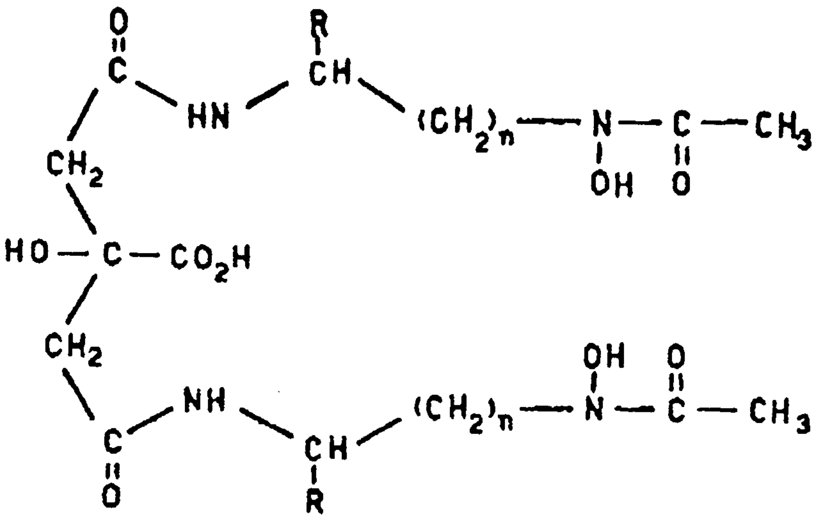

(X)

notamment la schizokinène (où R = -H et n = 2), l'aérobactine (où R = -COOH et n = 4) et l'arthrobactine (où R = -H et n = 4) (réf. 9).

Le procédé de l'invention est donc également fonctionnel avec des produits complexant spécifiquement les ions ferriques $Fe^{+++}$.

De tels produits sont également utilisables en condition anaérobie et/ou micro-aérophile.

En effet, dans le cas où fer est présent en condition anaérobie et/ou micro-aérophile sous forme d'ions $Fe^{++}$ et $Fe^{+++}$ en équilibre, l'addition d'un produit complexant les ions $Fe^{+++}$ déplacel'équilibre. Il en résulte un oxydation des ions $Fe^{++}$ en ions $Fe^{+++}$.

A mesure que les ions $Fe^{+++}$ sont complexés, la concentration en ions $Fe^{++}$ diminue. En ajoutant au milieu une quantité suffisante en produit complexant, on peut aboutir à la chélation de la quasi-totalité des ions $Fe^{+++}$ et $Fe^{++}$, après transformation de ces derniers en ions $Fe^{+++}$.

Le procédé de l'invention utilisant un produit complexant les ions ferriques $Fe^{+++}$ est donc également utilisable en condition anaérobie et/ou micro-aérophile.

Dans l'une ou l'autre des variantes d'utilisation de l'invention, c'est-à-dire en milieu anaérobie ou aérobie, le procédé de l'invention permet la régulation et le contrôle des différentes fermentations dont il a été question plus haut.

En effet, le procédé selon l'invention n'est pas irréversible. L'arrêt de l'addition du produit complexant, ou la réintroduction du fer dans le milieu pendant le déroulement des processus de fermentations, entraîne une reprise de la croissance des micro-organismes auparavant inhibée.

Il conviendra pour l'homme du métier de dèterminer, par des essais de routine, la concentration optimale du produit à ajouter.

En effet, il n'est pas nécessaire de complexer la totalité du fer présent dans le milieu de fermentation.

Il peut suffire de maintenir le fer disponible pour les micro-organismes à un niveau suffisamment bas

pour que le développement desdits micro-organismes ne présente pas de danger pour la fabrication, la conservation ou la consommation de ces produits alimentaires.

Selon un exemple préféré de mise en oeuvre de l'ivention, le pourcentage de fer présent à l'état complexé dans le substrat de fermentations mixtes anaérobies et/ou aérobies, varie entre environ 5 % et environ 100 %, du fer présent dans le milieu de fermentation.

Avantageusement, ce pourcentage varie entre environ 20 % et environ 100 %, du fer présent dans le milieu de fermentation.

En d'autres termes, le pourcentage de fer, encore disponible pour les micro-organismes, dans le milieu de fermentations mixtes anaérobies et/ou aérobies, varie entre 0 % et environ 95 %, avantageusement entre environ 0 % et environ 80 %, du fer présent dans le milieu de fermentation.

L'homme du métier pourra déterminer la concentration optimale en produit complexant le fer, en fonction des différents domaines d'utilisation de l'invention.

En effet, la présence de fer varie dans de grandes proportions suivant les organismes.

Il est en général présent en quantités relativement importantes dans les fourrages ensilés.

Au contraire, dans les industries des produits laitiers, le fer est souvent présent en quantités très faibles.

Des avantages et caractéristiques supplémentaires de l'invention apparaîtront encore à la lumière de la description plus détaillée qui suit, d'expériences mettant en oeuvre l'invention ainsi que des figures qui s'y rattachent et dans lesquelles :

- les figures 1a et 1b représentent les courbes de croissance de Lactobacillus plantarum et Streptococcus fecalis respectivement, en fonction de différentes concentrations de 2-2'-dipyridyl,
- les figures 2a et 2b représentent les courbes de pH obtenues pour ces mêmes bactéries lactiques et dans les mêmes conditions,
- les figures 3a et 3b représentent la courbe de production d'acide lactique pour ces mêmes bactéries et dans les mêmes conditions,
- la figure 4 représente la courbe de croissance de Clostridium perfringens en fonction de différentes concentrations de 2-2'-dipyridyl,
- la figure 5 représente les courbes de production d'acides lactique, acétique et butyrique chez Lactobacillus plantarum et Clostridium butyricum, en fonction de différentes concentrations de 2-2' -dipyridyl.

Dans les expériences qui suivent, on utilise des souches Clostridia (BC 8, BC 9, BC 11) isolées à partir de fourrage ensilé. Les résultats expérimentaux, bien qu'obtenus en laboratoire, peuvent donc être considérés comme très proches des conditions naturelles de fermentation, sans effectuer un extrapolation excessive.

Effet d'un agent complexant (2-2' -dipyridyl) sur différentes souches de Clostridia

Le milieu utilisé dans les tests qui suivent est un milieu BHI (Brain Heart Infusion), (Difco Products).

La température d'incubation est de 37 ° C.

Le tableau I ci-après résume les résultats obtenus après un jour d'incubation. La période d'incubation a été maintenue pendant 6 jours, mais les résultats n'ont pas varié pendant ce temps.

On voit d'après ce tableau que la croissance de Clostridia commence à être inhibée à partir d'une concentration en 2-2'-dipyridyl de 20 ppm dans le milieu BHI.

A 30 ppm, le développement est totalement stoppé dans le milieu BHI.

En réintroduisant du fer dans le milieu (par l'intermédiaire de sulfate $FeSO_4$), on constate une reprise de la croissance de Clostridia proche du niveau normal.

## TABLEAU I

### EFFETS DU 2-2'-DIPYRIDYL SUR LA CROISSANCE DE CLOSTRIDIA

| souche<br>dose (ppm) | BC 8 | BC 9 | BC 11 |
|---|---|---|---|
| 0 | +++ | +++ | +++ |
| 10 | +++ | +++ | +++ |
| 20 | + | + | + |
| 30 | - | - | - |
| 40 | - | - | - |
| 50 | - | - | - |
| 50 + $FeSO_4$ | +++(a) | +++ | +++(b) |

+++ = forte croissance
+ = croissance
- = pas de croissance
(a) = 1,4 mg $FeSO_4$ + 50 ppm de 2-2'-dipyridyl
(b) = 3,2 mg $FeSO_4$ + 50 ppm de 2-2'-dipyridyl
temps d'incubation = 6 jours.
ppm = partie par million.

Les figures 1 à 5, commentées ci-après, montrent les résultats obtenus avec différentes souches standard de micro-organismes, mises en incubation dans un milieu d'ensilage simulé tel que décrit par WOOLFORD M.K. et al, Journal of the Science of Food and Agriculture, (1974), 26, p 141-148, à une température de 33 °C.

Les figures 1a et 1b montrent que la croissance de Lactobacillus plantarum et Streptococcus fecalis - (bactéries lactiques) n'est pas affectée sous l'effet de diverses concentrations de 2-2'-dipyridyl (158 ppm et 312 ppm) par rapport à un milieu de référence ne contenant pas de 2-2'-dipyridyl.

De même, la chute du pH (figures 2a et 2b) et la quantité d'acide lactique (M.Z.) produit (figures 3a et 3b) ne sont pas sensiblement différentes dans un milieu contenant du 2-2'-dipyridyl à différentes concentrations et dans un milieu de référence dépourvu de 2-2'-dipyridyl.

Sur les figures 2 et 3, les concentrations utilisées en 2-2'dipyridyl sont les mêmes que sur la figure 1 et sont représentées par les mêmes courbes.

Il apparaît, au contraire, que le développement de Clostridia perfringens est fortement réduit en présence de 2-2'-dipyridyl à une concentration de 23 ppm (figure 4). En réintroduisant du fer dans le milieu contenant 23 ppm de 2-2'-dipyridyl, la croissance de Clostridia perfringens retrouve une évolution proche de celle obtenue dans un milieu dépourvu de 2-2'-dipyridyl.

La figure 5 montre la production d'acide lactique (M.Z), d'acide acétique (A.Z), et d'acide butyrique (B.Z), dans un milieu de fermentations mixtes comprenant Lactobacillus plantarum et Clostridium butyricum, en fonction de différentes concentrations en 2-2'-dipyridyl : 78 ppm, courbe (2), 156 ppm, courbe (3), et par rapport à une courbe de référence correspondant à un milieu dépourvu de 2-2'-dipyridyl, courbe (1).

Il apparaît que la production d'acides butyrique et acétique est stoppée en présence de 2-2'-dipyridyl alors que celle d'acide lactique est supérieure à la normale.

A partir d'un milieu de fermentations mixtes, l'addition de 2-2'-dipyridyl aboutit donc à un développement de la fermentation lactique au détriment des fermentations acétiques et butyriques.

Le procédé de l'invention peut être utilisé sous des formes très variées.

Il appartiendra à l'homme de métier de déterminer, par la simple mise en oeuvre d'essais de routine, les conditions d'utilisation optimales du procédé de l'invention en fonction de son domaine technique.

On peut, par exemple, dans le cas de la conservation du fourrage par ensilage, ajouter dans le silo un mélange formé des produits chélateurs du fer et des micro-organismes de fermentation, sous forme de poudre, éventuellement dissolvable dans un liquide.

On peut également répandre la poudre ou la solution sur l'herbe non encore coupée destinée à la production de fourrage ensilé.

Dans l'industrie fromagère, on peut, par exemple, ajouter le produit chélateur du fer pendant la fabrication du fromage. On peut également préparer à l'avance un mélange du produit chélateur et des microorganismes de fermentation et ajouter ce mélange au lait utilisé dans la fabrication des fromages.

On peut ainsi préparer un mélange, sous forme de poudre ou analogue, éventuellement dissolvable dans un liquide, et utilisable pour la régulation de fermentations mixtes, en vue notamment de la conservation de produits alimentaires, comprenant : un produit complexant le fer, notamment le 2-2'-dipyridyl, des micro-organismes de fermentation, notamment des bactéries lactiques, un produit de conservation n'altérant pas leurs propriétés respectives, tel que l'acide ascorbique, les anti-oxydants couramment utilisés dans la pratique.

L'homme de métier sera à même de déterminer, par la mise en oeuvre de simples essais de routine, si la présence d'un milieu de conservation sont nécessaires.

L'utilisation de l'invention a été, plus particulièrement envisagée, dans ce qui précède, pour les industries alimentaires.

D'autres industries pourront cependant tirer profit des avantages de l'invention. C'est le cas, notamment, des industries de recyclage du papier ou des déchets à base de papier.

C'est encore le cas de industries utilisant le sang comme matière première, notamment pour l' alimentation de l'homme et/ou de l'animal.

C'est encore le cas des industries du bois, du traitement d'excréments, ainsi que les industries des produits d'hygiène et d'entretien.

REFERENCES


1. Frederick ARCHIBALD,
FEMS Microbiology Letters,
19 (1983), 29-32.
2. L. MACHAM,
Process Biochemestry,
(Juin 1976), 12-17.
3. K. SHANKAR et al,
Journal of Bacteriology,
Vol. 63 (1952), 179-290.
4. Michael P. COUGHLAN,
Sci. Prog. Oxf.
(1971), 59, 1-23.
5. ZAEHNER H., KELLER-SHIERLEIN W., HUTTER R., HESS-LEISINGER K. et DEER A.,
Stoffwechselprodukte von Mikroorganismen : 40 ; Mitteilung Sideramine aus Aspergillaceeen Arch. Mikrobiol., 45, 119-135 (1963).
6. TEINTZE M., HOSSAIN M.B., BARNES C.L., LEONG J. et VAN DER HELM D.,
Structure of ferric pseudobatin, a siderophore from a plant growth promoting Pseudomonas.
Biochemistry, 20 : 6446-6456 (1981).
7. ROSENBERG H., et YOUNG I.G.,
Iron transport in the enteric bacteria. In :
Microbial iron metabolism. NEILANDS J.B. (Ed) Academic Press, New York and London (1974).
8. FOCHT D.D et VERSTRAETE W.,
Biochemical ecology of nitrification and denitrification, in :
Advances in Microbial Ecology, Vol. 1, p. 135-214, M. ALEXANDER (Ed) Plenum Press. New York and London (1977).
9. NEILANDS J.B.

Microbial iron compounds, Ann. Rev. Biochem., 50 : 715-731 (1981a).
10. FREDERICK C.B., BENTLEY M.D. et SHIVE W. Structure of triornicin, a new siderophore. Biochem., 20 : 2436-2438 (1981b).
11. FREDERICK C.B., BENTLEY M.D. et SHIVE W. Structure of the fungal siderophore, isotriornicin. Biochem. Biophys. Res. Commun., 105 : 133-139 (1982).
12. SNOW G.A., Mycobactins, Iron-chelating growth factors from Mycobacteria, Bacteriol. Rev., 34 : 100, (1970).

**Revendications**
**Revendications pour les Etats contractants suivants : BE, CH, DE, FR, GB, GR, IT, LI, LU, NL, SE**

1. Procédé de régulation et/ou de contrôle de fermentations mixtes anaérobies et/ou aérobies, au sein d'une composition contenant des bactéries lactiques, caractérisé par l'incorporation et le maintien au sein de la composition d'un produit choisi pour sa capacité à complexer spécifiquement le fer, notamment à l'état d'ions ferreux $Fe^{++}$ ou ferrique $Fe^{+++}$, et ce pour favoriser la fermentation lactique et, simultanément, éviter le développement au sein de ladite composition d'autres organismes pour lesquels le fer constitue un élément vital, le produit complexant du fer étant présent en une dose fonction du degré voulu de chélation du fer présent.

2. Procédé selon la revendication 1, caractérisé en ce que la composition est une composition alimentaire et que le produit chélatant le fer est choisi parmi tout produit naturel ou synthétique, non toxique, formant de manière spécifique des complexes stables avec le fer, notamment à l'état d'ions ferreux $Fe^{++}$ ou ferrique $Fe^{+++}$, au sein de ladite composition.

3. Procédé selon la revendication 1 ou 2, caractérisé en ce que le produit chélatant est un produit complexant le fer ferreux $Fe^{++}$.

4. Procédé selon la revendication 3, caractérisé en ce que le produit complexant est choisi parmi la ferrorosamine, la bathophénantroline, la 1,10-phénantroline, la sidérocholine A ou B.

5. Procédé selon l'une quelconque des revendications 1 à 3, caractérisé en ce que le produit complexant le fer est le 2-2'-dipyridyl.

6. Procédé selon la revendication 1 ou 2, caractérisé en ce que le produit chélatant est un produit complexant le fer ferrique $Fe^{+++}$.

7. Procédé selon la revendication 6, caractérisé en ce que le produit complexant le fer ferrique $Fe^{+++}$ est choisi parmi les phénolates-catécholates, tels que l'entérochéline ; les dihydroxamates, tels que les acides rhodoturulique et dimérumique ; les trihydroxamates tels que les ferrichromes, le coprogène, la triornicine, l'isotriornicine, les fusarinines, les ferrioxamines ; la parabactine, l'agrobactine, la mycobactine, la schizokinène, l'aérobactine, la pseudobactine, l'arthrobactine ; l'entérobactine ; l'EDDA ; la 8-hydroryquinoline.

8. Procédé selon l'une quelconque des revendications 1 à 7, caractérisé en ce que le pourcentage de fer présent à l'état complexé dans le substrat varie entre environ 5 % et environ 100 % du fer présent dans le milieu de fermentation, avantageusement varie entre environ 20 % et environ 100%.

9. Utilisation du procédé selon l'une quelconque des revendications 1 à 8, pour la régulation et/ou le contrôle de fermentations mixtes aérobies et/ou anaérobies dans des produits industriels mettant en jeu des produits organiques, dans lesquels des fermentations se produisent spontanément ou sont provoquées.

10. Mélange sous forme de poudre ou analogue, éventuellement dissolvable dans un liquide, utilisable pour la régulation et/ou le contrôle de fermentations mixtes aérobies et/ou anaérobies, au sein d'une composition, caractérisé en ce qu'il comprend :

- un produit complexant spécifiquement le fer, ce produit étant choisi parmi tout produit naturel ou synthétique formant de manière spécifique des complexes stables avec le fer, à l'état d'ions ferreux $Fe^{++}$ ou ferrique $Fe^{+++}$, au sein de ladite composition,
- des micro-organismes de fermentation, notamment des bactéries lactiques,
- un produit de conservation non toxique.

**11.** Mélange selon la revendication 10, caractérisé en ce qu'il est destiné à la régulation et/ou au contrôle de fermentations mixtes aérobies et/ou anaréobies au sein d'une composition alimentaire et que le produit complexant spécifique du fer et le produit de conservation sont tous deux non toxiques.

**12.** Mélange selon la revendication 10 ou 11, caractérisé en ce que le produit complexant le fer ferreux $Fe^{++}$ est choisi, notamment parmi la ferrorosamine, la bathophénantroline, la 1,10-phénantroline, la sidérocholine A ou B.

**13.** Mélange selon la revendication 10 ou 11, caractérisé en ce que le produit complexant le fer ferreux $Fe^{++}$, est avantageusement le 2-2'-dipyridyl.

**14.** Mélange selon la revendication 10 ou 11, caractérisé en ce que le produit complexant le fer ferrique $Fe^{++}$ est choisi notamment parmi les phénolates-catécholates, tels que l'entérochéline ; les dihydroxamates, tels que les acides rhodoturulique et dimérumique ; les trihydroxamates tels que les ferrichromes, le coprogène, la triornicine, l'isotriornicine, les fusarinines, les ferrioxamines ; la parabactine, l'agrobactine, la mycobactine, la schizokinène, l'aérobactine, la pseudobactine, l'arthrobactine ; l'entérobactine ; l'EDDA ; la 8-hydroxyquinoline.

**Revendications pour les Etats contractants suivants : AT, ES**

**1.** Procédé de régulation et/ou de contrôle de fermentations mixtes anaérobies et/ou aérobies, au sein d'une composition contenant des bactéries lactiques, caractérisé par l'incorporation et le maintien au sein de la composition d'un produit choisi pour sa capacité à complexer spécifiquement le fer, notamment à l'état d'ions ferreux $Fe^{++}$ ou ferrique $Fe^{+++}$, et ce pour favoriser la fermentation lactique et, simultanément, éviter le développement au sein de ladite composition d'autres organismes pour lesquels le fer constitue un élément vital, le produit complexant du fer étant présent en une dose fonction du degré voulu de chélation du fer présent.

**2.** Procédé selon la revendication 1, caractérisé en ce que la composition est une composition alimentaire et que le produit chélatant le fer est choisi parmi tout produit naturel ou synthétique, non toxique, formant de manière spécifique des complexes stables avec le fer, notamment à l'état d'ions ferreux $Fe^{++}$ ou ferrique $Fe^{+++}$, au sein de ladite composition.

**3.** Procédé selon la revendication 1 ou 2, caractérisé en ce que le produit chélatant est un produit complexant le fer ferreux $Fe^{++}$.

**4.** Procédé selon la revendication 3, caractérisé en ce que le produit complexant est choisi parmi la ferrorasamine, la bathophénantroline, la 1,10-phénantroline, la sidérocholine A ou B.

**5.** Procédé selon l'une quelconque des revendications 1 à 3, caractérisé en ce que le produit complexant le fer est le 2-2'-dipyridyl.

**6.** Procédé selon la revendication 1 ou 2, caractérisé en ce que le produit chélatant est un produit complexant le fer ferrique $Fe^{+++}$.

**7.** Procédé selon la revendication 6, caractérisé en ce que le produit complexant le fer ferrique $Fe^{+++}$ est choisi parmi les phénolates-catécholates, tels que l'entérochéline ; les dihydroxamates, tels que les acides rhodoturulique et dimérumique ; les trihydroxamates tels que les ferrichromes, le coprogène, la triornicine, l'isotriornicine, les fusarinines, les ferrioxamines ; la parabactine, l'agrobactine, la mycobactine, la schizokinène, l'aérobactine, la pseudobactine, l'arthrobactine ; l'entérobactine ; l'EDDA ; la 8-hydroxyquinoline.

**8.** Procédé selon l'une quelconque des revendications 1 à 7, caractérisé en ce que le pourcentage de fer présent à l'état complexé dans le substrat varie entre environ 5 % et environ 100 % du fer présent dans le milieu de fermentation, avantageusement varie entre environ 20 % et environ 100%.

**9.** Utilisation du procédé selon l'une quelconque des revendications 1 à 8, pour la régulation et/ou le contrôle de fermentations mixtes aérobies et/ou anaérobies dans des produits industriels mettant en jeu des produits organiques, dans lesquels des fermentations se produisent spontanément ou sont provoquées.

**10.** Procédé d'obtention d'un mélange sous forme de poudre ou analogue, éventuellement dissolvable dans un liquide, utilisable pour la régulation et/ou le contrôle de fermentations mixtes aérobies et/ou anaérobies, caractérisé en ce qu'il comprend le mélange entre :

- un produit complexant spécifiquement le fer, ce produit étant choisi parmi tout produit naturel ou synthétique, formant de manière spécifique des complexes stables avec le fer, à l'état d'ions ferreux $Fe^{++}$ ou ferrique $Fe^{+++}$, au sein d'une composition,
- des micro-organismes de fermentation, notamment des bactéries lactiques, et
- un produit de conservation non toxique.

**11.** Procédé d'obtention d'un mélange selon la revendication 10, caractérisé en ce que le mélange est destiné à la régulation et/ou au contrôle de fermentations mixtes aérobies et/ou anaréobies au sein d'une composition alimentaire et que le produit complexant spécifique du fer et le produit de conservation sont tous deux non toxiques.

**12.** Procédé d'obtention d'un mélange selon la revendication 10 ou 11, caractérisé en ce que le produit complexant le fer ferreux $Fe^{++}$ est choisi, notamment parmi la ferrorosamine, la bathophénantroline, la 1,10-phénantroline, la sidérocholine A ou B.

**13.** Procédé d'obtention d'un mélange selon la revendication 10 ou 11, caractérisé en ce que le produit complexant le fer ferreux $Fe^{++}$, est avantageusement le 2-2'-dipyridyl.

**14.** Procédé d'obtention d'un mélange selon la revendication 10 ou 11, caractérisé en ce que le produit complexant le fer ferrique $Fe^{+++}$ est choisi notamment parmi les phénolates-catécholates, tels que l'entérochéline ; les dihydroxamates, tels que les acides rhodoturulique et dimérumique ; les trihydroxamates tels que les ferrichromes, le coprogène, la triornicine, l'isotriornicine, les fusarinines, les ferrioxamines ; la parabactine, l'agrobactine, la mycobactine, la schizokinène, l'aérobactine, la pseudobactine, l'arthrobactine ; l'entérobactine ; l'EDDA ; la 8-hydroxyquinoline.

**Claims**

**Claims for the following Contracting States : BE, CH, DE, FR, GB, GR, IT, LI, LU, NL, SE**

**1.** Process for the regulation and/or control of anaerobic and/or aerobic mixed fermentations within a composition containing lactic acid bacteria, characterized by the incorporation and the maintenance within the composition of a product selected for its capacity to complex iron specifically, in particular in the state of ferrous $Fe^{++}$ or ferric $Fe^{+++}$ ions, and to do so in order to promote lactic acid fermentation and, simultaneously, to prevent the growth within the said composition of other organisms for which iron constitutes a vital element, the complexing product for iron being present in a dose which is a function of the desired degree of chelation of the iron present.

**2.** Process according to Claim 1, characterized in that the composition is a dietary composition and that the product chelating the iron is selected from any non-toxic, synthetic or natural product forming specifically stable complexes with iron, in particular in the state of ferrous $Fe^{++}$ or ferric $Fe^{+++}$ ions within the said composition.

**3.** Process according to Claim 1 or 2, characterized in that the chelating product is a product complexing ferrous iron $Fe^{++}$.

**4.** Process according to Claim 3, characterized in that the complexing product is selected from ferrorosamine, bathophenanthroline, 1,10-phenanthroline, siderocholine A or B.

**5.** Process according to any one of the Claims 1 to 3, characterized in that the product complexing iron is 2,2'-dipyridyl.

**6.** Process according to Claim 1 or 2, characterized in that the chelating product is a product complexing ferric iron $Fe^{+++}$.

**7.** Process according to Claim 6, characterized in that the product complexing ferric iron $Fe^{+++}$ is selected from the phenolates-catecholates, such as enterochelin; the dihydroxamates, such as rhodoturulic and dimerumic acids; the trihydroxamates such as the ferrichromes, coprogen, triornicin, isotriornicin, the fusarinins, the ferrioxamines; parabactin, agrobactin, mycobactin, schizokinene, aerobactin, pseudobactin, arthrobactin; enterobactin; EDDA; 8-hydroxyquinoline.

**8.** Process according to any one of the Claims 1 to 7, characterized in that the percentage of iron present in the complexed state in the substrate varies between about 5% and about 100% of the iron present in the fermentation medium, and advantageously varies between about 20% and about 100%.

**9.** Utilization of the process according to any one of the Claims 1 to 8 for the regulation and/or control of aerobic and/or anaerobic mixed fermentations in industrial products making use of organic substances in which fermentations occur spontaneously or are induced.

**10.** Mixture in the form of a powder or something similar, which can possibly be dissolved in a liquid, and used for the regulation and/or control of aerobic and/or anaerobic mixed fermentations within a composition, characterized in that it comprises:

- a product complexing iron specifically, this product being selected from any synthetic or natural product forming specifically stable complexes with iron in the state of ferrous $Fe^{++}$ or ferric $Fe^{+++}$ ions, within the said composition,
- micro-organisms of fermentation, in particular lactic acid bacteria,
- a non-toxic preservative.

**11.** Mixture according to Claim 10, characterized in that it is intended for the regulation and/or control of aerobic and/or anaerobic mixed fermentations within a dietary composition and the complexing product specific for iron and the preservative are both non toxic.

**12.** Mixture according to Claim 10 or 11, characterized in that the product complexing ferrous iron $Fe^{++}$ is selected in particular from ferrorosamine, bathophenanthroline, 1,10-phenanthroline, siderocholine A or B.

**13.** Mixture according to Claim 10 or 11, characterized in that the product complexing ferrous iron $Fe^{++}$ is advantageously 2,2'-dipyridyl.

**14.** Mixture according to Claim 10 or 11, characterized in that the product complexing ferric iron $Fe^{+++}$ is selected in particular from the phenolates-catecholates, such as enterochelin; the dihydroxamates, such as the rhodoturulic and dimerumic acids; the trihydroxamates such as the ferrichromes, coprogen, triornicin, isotriornicin, the fusarinins, the ferrioxamines; parabactin, agrobactin, mycobactin, schizokinene, aerobactin, pseudobactin, arthrobactin; enterobactin; EDDA; 8-hydroxyquinoline.

**Claims for the following Contracting States : AT, ES**

**1.** Process for the regulation and/or control of anaerobic and/or aerobic mixed fermentations within a composition containing lactic acid bacteria, characterized by the incorporation and the maintenance within the composition of a product selected for its capacity to complex iron specifically, in particular in the state of ferrous $Fe^{++}$ or ferric $Fe^{+++}$ ions, and to do so in order to promote lactic acid fermentation and, simultaneously, to prevent the growth within the said composition of other organisms for which iron constitutes a vital element, the complexing product for iron being present in a dose which is a function of the desired degree of chelation of the iron present.

**2.** Process according to Claim 1, characterized in that the composition is a dietary composition and that the product chelating the iron is selected from any non-toxic, synthetic or natural product forming

specifically stable complexes with iron, in particular in the state of ferrous $Fe^{++}$ or ferric $Fe^{+++}$ ions within the said composition.

**3.** Process according to Claim 1 or 2, characterized in that the chelating product is a product complexing ferrous iron $Fe^{++}$.

**4.** Process according to Claim 3, characterized in that the complexing product is selected from ferrorosamine, bathophenanthroline, 1,10-phenanthroline, siderocholine A or B.

**5.** Process according to any one of the Claims 1 to 3, characterized in that the product complexing iron is 2,2'-dipyridyl.

**6.** Process according to Claim 1 or 2, characterized in that the chelating product is a product complexing ferric iron $Fe^{+++}$.

**7.** Process according to Claim 6, characterized in that the product complexing ferric iron $Fe^{+++}$ is selected from the phenolates-catecholates, such as enterochelin; the dihydroxamates, such as rhodoturulic and dimerumic acids; the trihydroxamates such as the ferrichromes, coprogen, triornicin, isotriornicin, the fusarinins, the ferrioxamines; parabactin, agrobactin, mycobactin, schizokinene, aerobactin, pseudobactin, arthrobactin; enterobactin; EDDA; 8-hydroxyquinoline.

**8.** Process according to any one of the Claims 1 to 7, characterized in that the percentage of iron present in the complexed state in the substrate varies between about 5% and about 100% of the iron present in the fermentation medium, and advantageously varies between about 20% and about 100%.

**9.** Utilization of the process according to any one of the claims 1 to 8 for the regulation and/or control of aerobic and/or anaerobic mixed fermentations in industrial products making use of organic substances in which fermentations occur spontaneously or are induced.

**10.** Process for the preparation of a mixture in the form of a powder or something similar, which can possibly be dissolved in a liquid, and used for the regulation and/or control of aerobic and/or anaerobic mixed fermentations within a composition, characterized in that it comprises mixing :

- a product complexing iron specifically, this product being selected from any synthetic or natural product forming specifically stable complexes with iron in the state of ferrous $Fe^{++}$ or ferric $Fe^{+++}$ ions, within the said composition,
- micro-organisms of fermentation, in particular lactic acid bacteria,
- a non-toxic preservative.

**11.** Process for the preparation of a mixture according to claim 10, characterized in that the mixture is intented for the regulation and/or control of aerobic and/or anaerobic mixed fermentations within fermentations within a dietary composition and the complexing product specific for iron and the preservative are both non toxic.

**12.** Process for the preparation of a mixture according to claim 10 or 11, characterized in that the product complexing ferrous iron $Fe^{++}$ is selected in particular from ferrorosamine, bathophenanthroline, 1,10-phenanthroline, siderocholine A or B.

**13.** Process for the preparation of a mixture to claim 10 or 11, characterized in the the product complexing ferrous iron $Fe^{++}$ is advantageously 2,2'-dypyridyl.

**14.** Process for the preparation of a mixture according to claim 10 or 11, characterized in that the product complexing ferric iron $Fe^{+++}$ is selected in particular from the phenolates-catecholates, such as enterochelin; the dihydroxamates, such as the rhodoturulic and dimerumic acids ; the trihydroxamates such as the ferrichromes, coprogen, triornicin, isotriornicin, the fusarinins, the ferrioxamines ; parabactin, agrobactin, mycobactin, schizokinene, aerobactin, pseudobactin, arthrobactin ; enterobactin ; EDDA ; 8-hydroxyquinoline.

**Patentansprüche**

**Patentansprüche für folgende Vertragsstaaten : BE, CH, DE, FR, GB, GR, IT, LI, LU, NL, SE**

1. Verfahren zur Regulation und/oder Kontrolle von anaeroben und/oder aeroben Mischfermentationen innerhalb einer Zusammensetzung, die Milchsäurebakterien enthält, gekennzeichnet durch den Einbau und die Aufrechterhaltung eines Produktes innerhalb der Zusammensetzung, das nach seiner Fähigkeit zur spezifischen Komplexierung von Eisen ausgewählt ist, insbesondere im Ionenzustand als $Fe^{++}$ oder $Fe^{+++}$, zur Begünstigung der Milchsäuregärung und gleichzeitig Vermeidung der Entwicklung von anderen Organismen innerhalb der Zusammensetzung, für die Eisen ein essentielles Element darstellt, wobei der Komplexbildner für Eisen in einer Menge vorliegt, die ausreicht, um das gewünschte Maß der Chelatisierung des vorhandenen Eisens zu erreichen.

2. Verfahren nach Anspruch 1, dadurch gekennzeichnet, daß die Zusammensetzung eine Nahrungsmittelzusammensetzung ist, und der Chelatbildner für das Eisen ausgewählt ist unter allen natürlichen oder synthetischen nicht-toxischen Produkten, die innerhalb der Zusammensetzung in spezifischer Weise stabile Komplexe mit dem Eisen bilden, insbesondere im $Fe^{++}$ oder $Fe^{+++}$-Zustand.

3. Verfahren nach Anspruch 1 oder 2, dadurch gekennzeichnet, daß der Chelatbildner ein Komplexbildner für $Fe^{++}$ ist.

4. Verfahren nach Anspruch 3, dadurch gekennzeichnet, daß der Komplexbildner ausgewählt ist aus Ferrorosamin, Bathophenantrolin, 1,10-Phenantrolin, Siderocholin A oder B.

5. Verfahren nach einem der Ansprüche 1 bis 3, dadurch gekennzeichnet, daß der Komplexbildner für Eisen 2-2'-Dipyridyl ist.

6. Verfahren nach Anspruch 1 oder 2, dadurch gekennzeichnet, daß der Chelatbildner ein Komplexbildner für $Fe^{+++}$ ist.

7. Verfahren nach Anspruch 6, dadurch gekennzeichnet, daß der Komplexbildner für $Fe^{+++}$ ausgewählt ist aus Phenolaten-Catecholaten, wie Enterochelin; Dihydroxamaten, wie Rhodoturulinsäure und Demeruminsäure; Trihydroxamaten, wie Ferrichromen, Coprogen, Triornicin, Isotriornicin, Fusarininen, Ferrioxaminen; Parabactin, Agrobactin, Mycobactin, Schizokinen, Aerobactin, Pseudobactin, Arthrobactin; Enterobactin; EDDA, 8-Hydroxychinolin.

8. Verfahren nach einem der Ansprüche 1 bis 7, dadurch gekennzeichnet, daß der Prozentsatz des im komplexierten Zustand vorliegenden Eisens im Substrat zwischen etwa 5 % und etwa 100 % des im Fermentationsmedium vorhandenen Eisens beträgt, vorzugsweise zwischen etwa 20 und etwa 100 %.

9. Verwendung des Verfahrens nach einem der Ansprüche 1 bis 8 für die Regulation und/oder die Kontrolle von aeroben und/oder anaeroben Mischfermentationen in industriellen Produkten, die organische Produkte aufweisen, in denen die Fermentationen spontan stattfinden oder veranlaßt werden.

10. Gemisch in Form eines Pulvers oder Analogs, gegebenenfalls lösbar in einer Flüssigkeit, verwendbar für die Regulation und/oder Kontrolle von aeroben und/oder anaeroben Mischfermentationen, innerhalb einer Zusammensetzung, dadurch gekennzeichnet, daß es umfaßt:
    - ein Produkt, das spezifische Eisen komplexiert, wobei das Produkt ausgewählt ist aus allen natürlichen oder synthetischen Produkten, die innerhalb der Zusammensetzung in spezifischer Weise stabile Komplexe mit Eisen bilden, im Ionenzustand als $Fe^{++}$ oder $Fe^{+++}$,
    - Fermentationsmikroorganismen, insbesondere Milchsäurebakterien,
    - ein nicht-toxisches Konservierungsprodukt.

11. Gemisch nach Anspruch 10, dadurch gekennzeichnet, daß es für die Regulation und/oder Kontrolle von aeroben und/oder anaeroben Mischfermentationen innerhalb einer Nahrungsmittelzusammensetzung bestimmt ist und daß das spezifisch Eisen komplexierende Produkt und das Konservierungsprodukt beide nicht-toxisch sind.

12. Gemisch nach Anspruch 10 oder 11, dadurch gekennzeichnet, daß der Komplexbildner für das Eisen im $Fe^{++}$-Zustand ausgewählt ist aus insbesondere Ferrorosamin, Bathophenantrolin, 1,10-Phenantrolin,

Siderocholin A oder B.

**13.** Gemisch nach Anspruch 10 oder 11, dadurch gekennzeichnet, daß der Komplexbildner für das Eisen im $Fe^{++}$-Zustand bevorzugt 2-2'-Dipyridyl ist.

**14.** Gemisch nach Anspruch 10 oder 11, dadurch gekennzeichnet, daß der Komplexbildner für das Eisen im $Fe^{+++}$-Zustand ausgewählt ist insbesondere aus Phenolaten-Catecholaten, wie Enterochelin; Dihydroxamaten, wie Rhodoturulinsäuren und Demeruminsäuren; Trihydroxamaten, wie Ferrichromen, Coprogen, Triornicin, Isotriornicin, Fusarinin, Ferrioxaminen; Parabactin, Agrobactin, Mycobactin, Schizokinen, Aerobactin, Pseudobactin, Arthrobactin, Enterobactin; EDDA, 8-Hydroxychinolin.

**Patentansprüche für folgende Vertragsstaaten : AT, ES**

**1.** Verfahren zur Regulation und/oder Kontrolle von anaeroben und/oder aeroben Mischfermentationen innerhalb einer Zusammensetzung, die Milchsäurebakterien enthält, gekennzeichnet durch den Einbau und die Aufrechterhaltung eines Produktes innerhalb der Zusammensetzung, das nach seiner Fähigkeit zur spezifischen Komplexierung von Eisen ausgewählt ist, insbesondere im Ionenzustand als $Fe^{++}$ oder $Fe^{+++}$, zur Begünstigung der Milchsäuregärung und gleichzeitig Vermeidung der Entwicklung von anderen Organismen innerhalb der Zusammensetzung, für die Eisen ein essentielles Element darstellt, wobei der Komplexbildner für Eisen in einer Menge vorliegt, die ausreicht, um das gewünschte Maß der Chelatisierung des vorhandenen Eisens zu erreichen.

**2.** Verfahren nach Anspruch 1, dadurch gekennzeichnet, daß die Zusammensetzung eine Nahrungsmittelzusammensetzung ist, und der Chelatbildner für das Eisen ausgewählt ist unter allen natürlichen oder synthetischen nicht-toxischen Produkten, die innerhalb der Zusammensetzung in spezifischer Weise stabile Komplexe mit dem Eisen bilden, insbesondere im $Fe^{++}$ oder $Fe^{+++}$-Zustand.

**3.** Verfahren nach Anspruch 1 oder 2, dadurch gekennzeichnet, daß der Chelatbildner ein Komplexbildner für $Fe^{++}$ ist.

**4.** Verfahren nach Anspruch 3, dadurch gekennzeichnet, daß der Komplexbildner ausgewählt ist aus Ferrorosamin, Bathophenantrolin, 1,10-Phenantrolin, Siderocholin A oder B.

**5.** Verfahren nach einem der Ansprüche 1 bis 3, dadurch gekennzeichnet, daß der Komplexbildner für Eisen 2-2'-Dipyridyl ist.

**6.** Verfahren nach Anspruch 1 oder 2, dadurch gekennzeichnet, daß der Chelatbildner ein Komplexbildner für $Fe^{+++}$ ist.

**7.** Verfahren nach Anspruch 6, dadurch gekennzeichnet, daß der Komplexbildner für $Fe^{+++}$ ausgewählt ist aus Phenolaten-Catecholaten, wie Enterochelin; Dihydroxamaten, wie Rhodoturulinsäure und Demeruminsäure; Trihydroxamaten, wie Ferrichromen, Coprogen, Triornicin, Isotriornicin, Fusarininen, Ferrioxaminen; Parabactin, Agrobactin, Mycobactin, Schizokinen, Aerobactin, Pseudobactin, Arthrobactin; Enterobactin; EDDA, 8-Hydroxychinolin.

**8.** Verfahren nach einem der Ansprüche 1 bis 7, dadurch gekennzeichnet, daß der Prozentsatz des im komplexierten Zustand vorliegenden Eisens im Substrat zwischen etwa 5 % und etwa 100 % des im Fermentationsmedium vorhandenen Eisens beträgt, vorzugsweise zwischen etwa 20 und etwa 100 %.

**9.** Verwendung des Verfahrens nach einem der Ansprüche 1 bis 8 für die Regulation und/oder die Kontrolle von aeroben und/oder anaeroben Mischfermentationen in industriellen Produkten, die organische Produkte aufweisen, in denen die Fermentationen spontan stattfinden oder veranlaßt werden.

**10.** Verfahren zur Herstellung eines Gemisches in Form eines Pulvers oder Analogs, gegebenenfalls lösbar in einer Flüssigkeit, verwendbar für die Regulation und/oder Kontrolle von aeroben und/oder anaeroben Mischfermentationen, innerhalb einer Zusammensetzung, dadurch gekennzeichnet, daß das Gemisch umfaßt:

- ein Produkt, das spezifische Eisen komplexiert, wobei das Produkt ausgewählt ist aus allen

natürlichen oder synthetischen Produkten, die innerhalb der Zusammensetzung in spezifischer Weise stabile Komplexe mit Eisen bilden, im Ionenzustand als $Fe^{++}$ oder $Fe^{+++}$,

- Fermentationsmikroorganismen, insbesondere Milchsäurebakterien,
- ein nicht-toxisches Konservierungsprodukt.

**11.** Verfahren zur Herstellung eines Gemisches nach Anspruch 10, dadurch gekennzeichnet, daß das Gemisch für die Regulation und/oder Kontrolle von aeroben und/oder anaeroben Mischfermentationen innerhalb einer Nahrungsmittelzusammensetzung bestimmt ist und daß das spezifisch Eisen komplexierende Produkt und das Konservierungsprodukt beide nicht-toxisch sind.

**12.** Verfahren zur Herstellung eines Gemisches nach Anspruch 10 oder 11, dadurch gekennzeichnet, daß der Komplexbildner für das Eisen im $Fe^{++}$-Zustand ausgewählt ist aus insbesondere Ferrorosamin, Bathophenantrolin, 1,10-Phenantrolin, Siderocholin A oder B.

**13.** Verfahren zur Herstellung eines Gemisches nach Anspruch 10 oder 11, dadurch gekennzeichnet, daß der Komplexbildner für das Eisen im $Fe^{++}$-Zustand bevorzugt 2-2'-Dipyridyl ist.

**14.** Verfahren zur Herstellung eines Gemisches nach Anspruch 10 oder 11, dadurch gekennzeichnet, daß der Komplexbildner für das Eisen im $Fe^{+++}$-Zustand ausgewählt ist insbesondere aus Phenolaten-Catecholaten, wie Enterochelin; Dihydroxamaten, wie Rhodoturulinsäuren und Demeruminsäuren; Trihydroxamaten, wie Ferrichromen, Coprogen, Triornicin, Isotriornicin, Fusarinin, Ferrioxaminen; Parabactin, Agrobactin, Mycobactin, Schizokinen, Aerobactin, Pseudobactin, Arthrobactin, Enterobactin; EDDA, 8-Hydroxychinolin.

Figures 1

Courbe de croissance mesurée par densité optique (D.O)

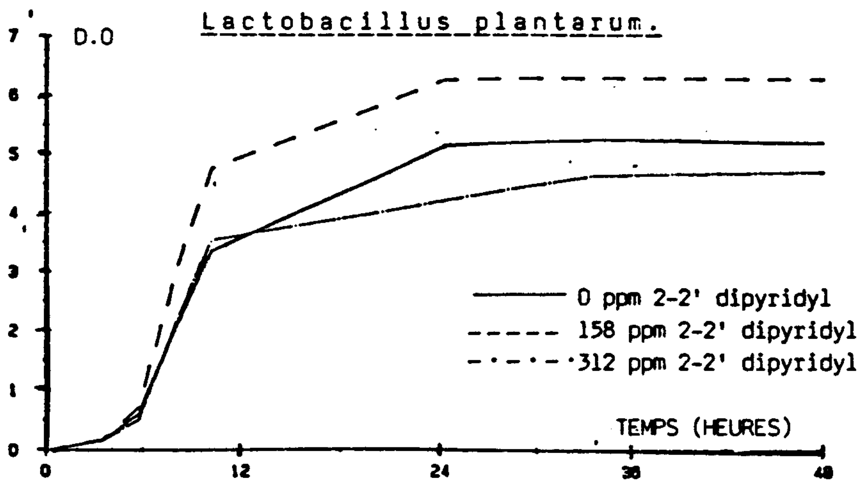


figure 1a

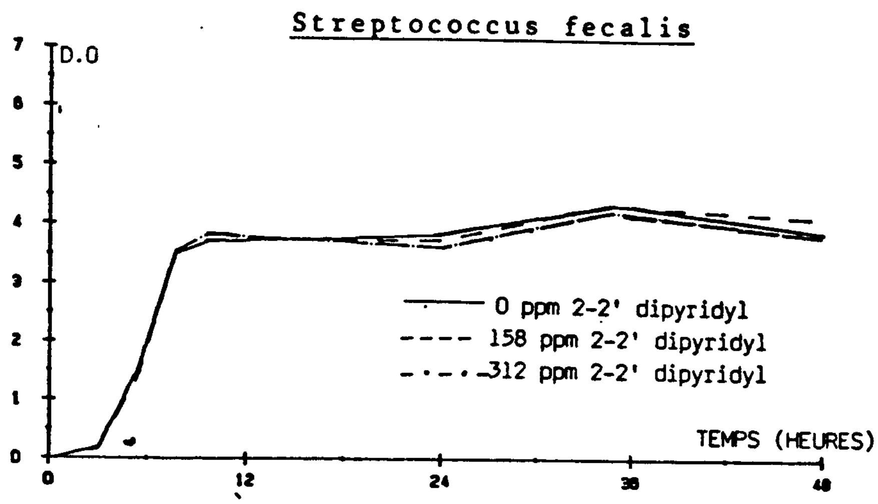


figure 1B

Figures 2

pH

Lactobacillus plantarum.

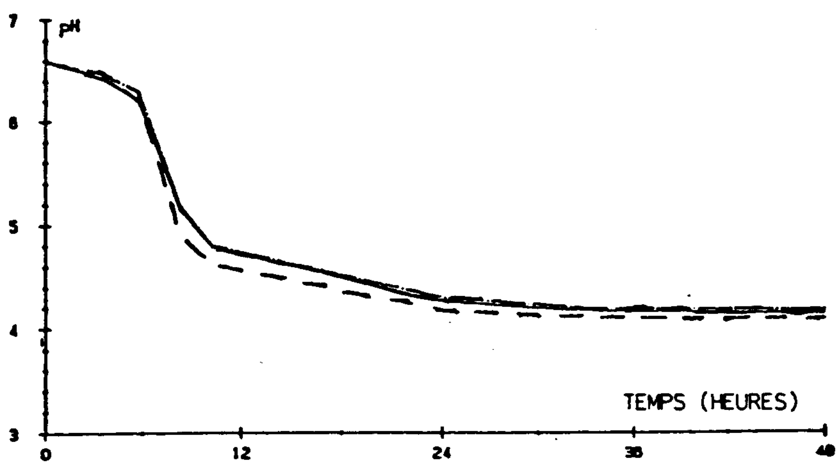


(figure 2a)

Streptococcus fecalis.

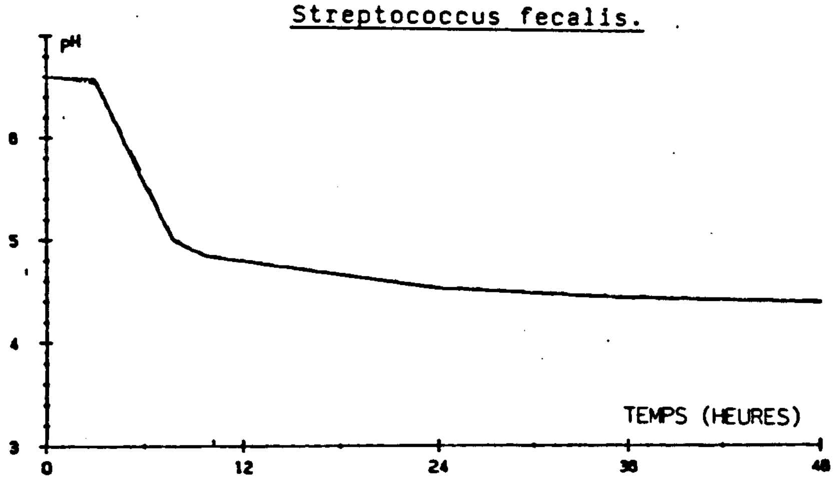


(figure 2b)

Figures 3

Production d'acide lactique

Lactobacillus plantarum.

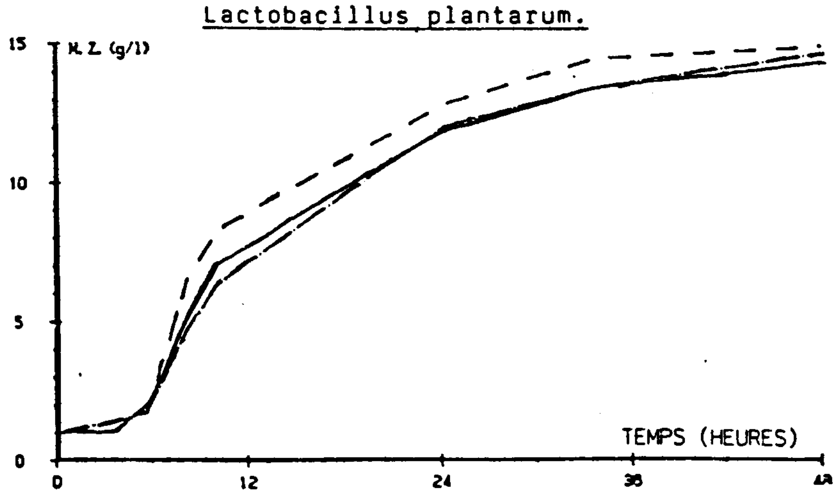


(figure 3a)

Streptococcus fecalis.

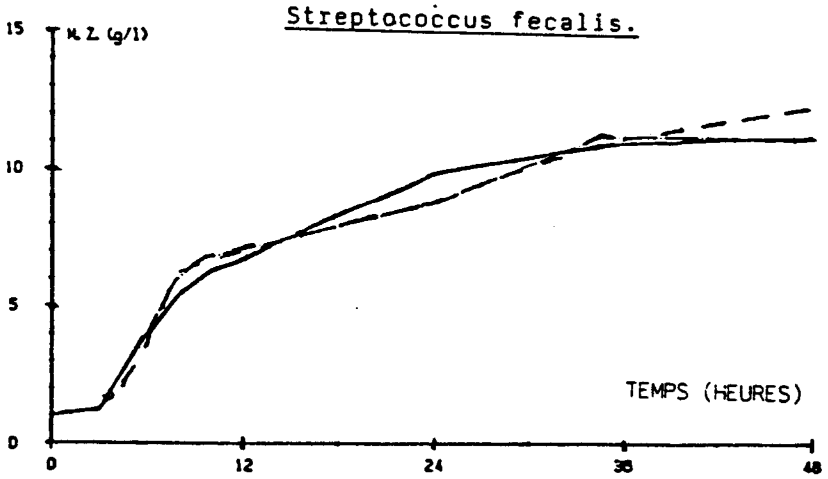


(figure 3b)

Figure 4

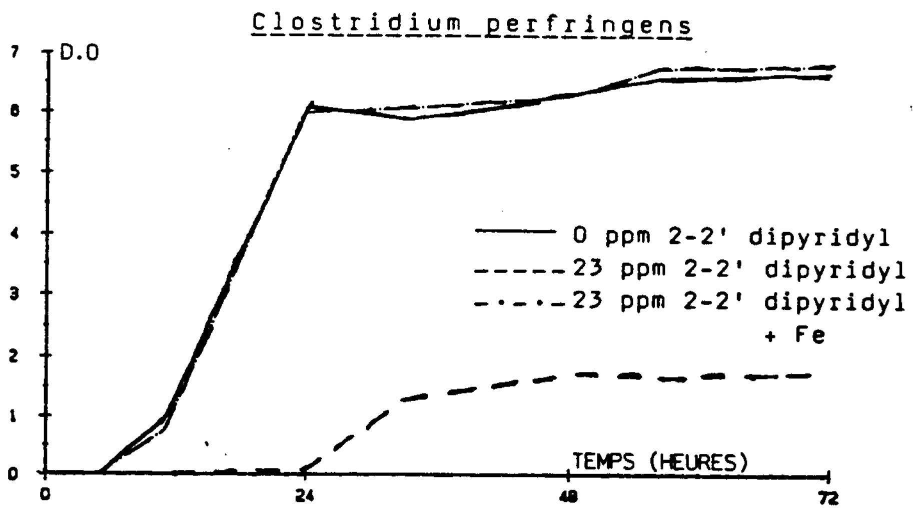


Courbe de croissance mesurée par densité optique (D.O)

Figure 5

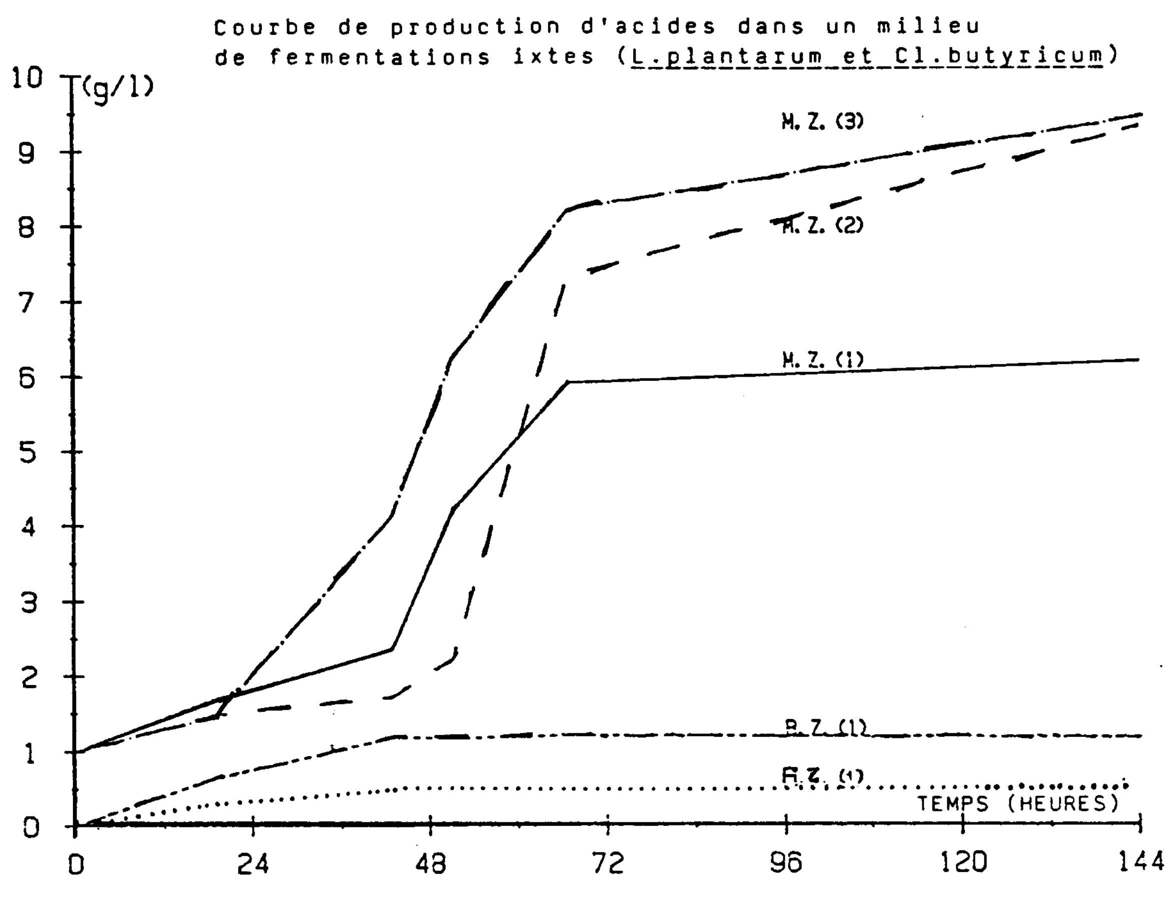
Courbe de production d'acides dans un milieu
de fermentations ixtes (L.plantarum et Cl.butyricum)
(g/l)
10
9
8
7
6
5
4
3
2
1
0
M. Z. (3)
M. Z. (2)
M. Z. (1)
R. Z. (1)
R. Z. (1)
TEMPS (HEURES)
0
24
48
72
96
120
144